Europäisches Patentamt

**European Patent Office**

**Office européen des brevets**

(19)

(11) Publication number: **0 037 867**
A1

# EUROPEAN PATENT APPLICATION

(12)

(21) Application number: **80301193.1**

(22) Date of filing: **15.04.80**

(51) Int. Cl.³: **G 01 N 33/46**, G 01 N 3/20

(43) Date of publication of application: **21.10.81**
**Bulletin 81/42**

(84) Designated Contracting States: **AT BE CH DE FR IT LI LU NL SE**

(71) Applicant: **Measuring and Process Control Limited, Phoenix House New Road, Rainham, Essex, RM13 8RJ (GB)**

(72) Inventor: **Serry, Victor, Flat 1 6 The Paragon, London. SE3 (GB)**

(74) Representative: **Smith, Martin Stanley, Stevens, Hewlett & Perkins 5, Quality Court Chancery Lane, London WC2A 1HZ (GB)**

(54) **Method of stress grading timber, and apparatus therefor.**

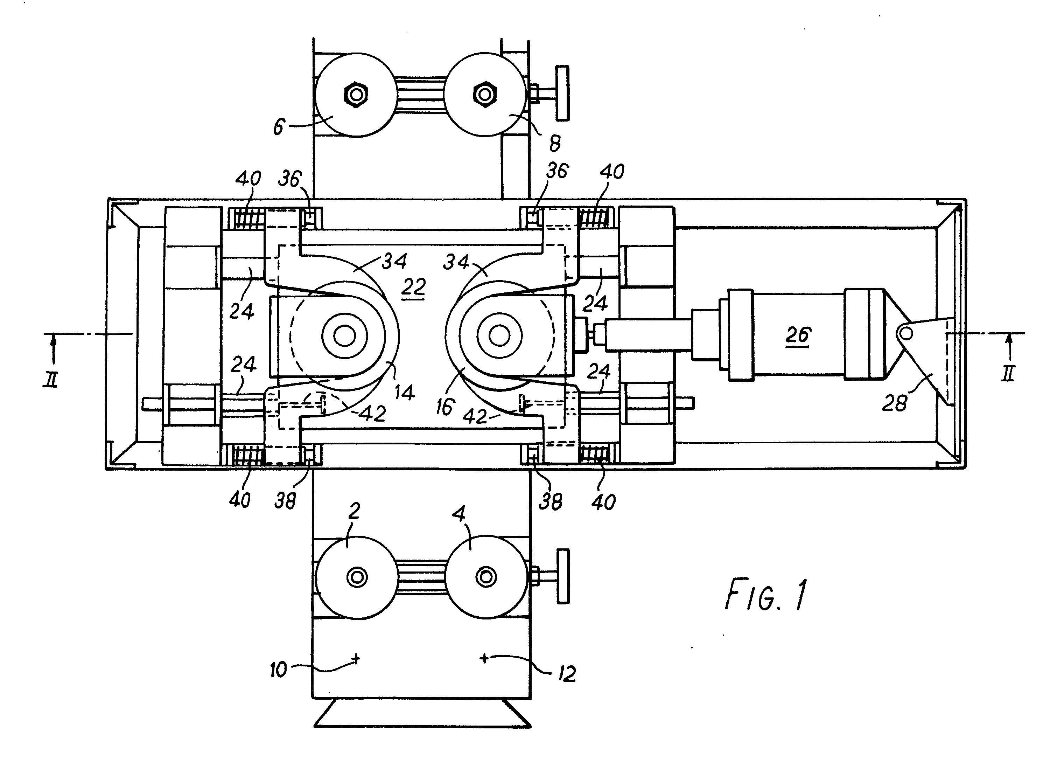

(57) A machine for stress grading of timber comprising fixed rollers (2, 4, 6 and 8) through which timber to be tested may pass, and movable rollers (14, 16) mounted on a trolley (22). The trolley (22) is movable in a lateral direction (from left to right in Figure 1) in order to bias one roller (14, 16) or the other against the timber as it passes through, thus applying a bending force to the timber. Once the timber has passed through, the machine in one direction, it is reversed and run back through the machine, the trolley (22), on this reverse traverse, being moved to apply the other roller (14, 16) to the timber, thus applying a bending force in the opposite direction.

- 1 -

"IMPROVEMENTS RELATING TO THE MECHANICAL STRESS
GRADING OF TIMBER"

It is known that the modulus of elasticity
of timber, as measured by non-destructive means, gives a
good indication of the mechanical properties of a piece
of timber.

A number of methods for grading square sawn
timber are known in which the modulus of elasticity is
repeatedly measured as a piece of timber is passed
lengthwise through a system of rollers which subject
the timber to a bending stress.

In one conventional arrangement the roller
system comprises two spaced abutment rollers which are
engaged by one side of the piece of timber and a load
roller, equidistant between the abutment rollers, which
applies a deflecting load to the opposite side of the
piece.

It is well known that measurements obtained
by passing timber through a roller system comprising
only three rollers arranged as described above do not
provide sufficient information because the initial
straightness of the piece is not usually known.   To
overcome this difficulty machines are provided with
two roller systems, arranged one after the other and
through which the timber passes lengthwise in succession.

Such a machine is quite large and its cost
is increased by the fact that each roller system requires
its own deflection measuring system.   Moreover it
requires the use of two men, one to feed the timber in
at one end and the other to remove the timber at the
other end.

These disadvantages are reduced and a more economical machine provided if in accordance with this invention the machine is arranged so that a piece of timber passes through it twice, once in one direction and once in the opposite direction. In this way only one measuring system is required while the machine can be operated by one man since the piece of timber is redelivered at the same end of the machine as it enters.

Thus in accordance with a first aspect of the invention there is provided a method of stress grading timber, said method comprising passing timber to be graded in a forward direction through a system of rollers whilst simultaneously causing said rollers to bend the timber in one direction, thence reversing the timber so that it passes back through the rollers in the reverse direction whilst simultaneously causing said rollers to bend the timber in the other direction, and monitoring the conditions under which the timber is bent so as to enable the timber to be graded.

In one form of machine for carrying out the above method, the three roller system above described is duplicated so as to provide two spaced pairs of abutment rollers and arranged centrally between them a pair of load rollers, the rollers of each pair being normally spaced apart by a distance somewhat greater than the width of the timber to be tested. The timber is bent as it passes through the roller system by one of the load rollers moving against the timber and bending it between the opposed abutment rollers. When the timber is reversed so that it passes back through the roller system in the opposite direction, the other of the load rollers moves against the timber and bends it between the other abutment rollers.

In accordance with a second aspect of the invention there is provided apparatus for the stress grading of timber, said apparatus comprising a series of rollers through which timber to be tested may pass, reversible drive means for moving the timber through the roller system, means for reversing the drive of said drive means after the timber has passed or almost passed through the roller system in a forward direction, means for causing said rollers to bend the timber in one direction as it passes through the roller system in the forward direction and in the opposite direction as the timber passes through the roller system in the reverse direction, and means for monitoring the conditions under which the timber is bent so as to enable the timber to be graded.

In a preferred embodiment of the invention the roller system comprises a minimum of six rollers arranged in two sets of three, each set comprising a pair of spaced abutment rollers and a load roller situated centrally between them, but arranged to be on the opposite side of the timber, when present. The two sets of rollers face one another on opposite sides of the timber, when present - in other words, each abutment roller of one set has an abutment roller of the other set facing it on the opposite side of the timber, and the load roller of each set faces the load roller from the other set on the opposite side of the timber. Means are provided whereby the pair of load rollers on the one hand and the two pairs of abutment rollers on the other hand may be moved relatively to one another in a direction

- 4 -                                      0037867

transverse to the direction of movement of the timber under test in such a manner that while the timber is passing through the machine in one direction one of the two load rollers presses the timber against and bends it between the opposed abutment rollers whereas when the timber is passing through the machine in the opposite direction it is the other load roller and the other two abutment rollers which act to bend the timber.

In one convenient arrangement the two load rollers are mounted in a carriage which is movable in the required direction under control of a hydraulic or pneumatic actuator, while the abutment rollers are held fixed.

In an alternative arrangement the axes of the load rollers are fixed while the abutment rollers are moved each by a respective hydraulic or pneumatic actuator transversely of the direction of movement of the timber, the extent of movement away from the path of the timber being limited by means of stops.

In both such arrangements and in order to move the timber through the machine the two load rollers may be driven while the four abutment rollers may be freely rotatable, but the reverse arrangement in which the abutment rollers are driven and the load rollers idle would be equally effective and have some advantages, but would be more costly.

Control of the movement of the timber may be effected by means of a sensor which is arranged to be effective to stop the machine when the trailing end of a piece of timber going into the machine almost reaches the leading pair of abutment rollers, and then to reverse

0037867

the machine and at the same time make the necessary changes in the loading arrangements.    Another sensor may be arranged to be effective to start the machine when the leading end of a piece of timber reaches the trailing pair of abutment rollers.

The manner in which the conditions of bending are monitored can vary.    In one method of measurement the deflection is fixed and the load required to produce such deflection is measured while in another method the load is fixed and the deflection produced by such load is measured.

It will be appreciated that the first of the above described arrangements may readily be operated under conditions in which either the load or the deflection is fixed and the other parameter is measured, or under conditions where both parameters are measured, whereas the second above-described arrangement is essentially appropriate for use where the deflection is fixed, although the extent of deflection can be altered by appropriate movement of the stops.

However both these methods of measurement as used in existing stress grading machines are defective in practice in that it is not possible under dynamic conditions to hold the parameters intended to be fixed within sufficiently close margins.

If the deflection is fixed then vibration variations in the size of the timber, and the sudden changes in load when the piece enters and leaves the roller system, all tend to create disturbances in deflection which are not taken into account in assessing the modulus of elasticity.

Similarly if the load is fixed then the sudden load change, vibrations caused by surface irregularities, and changes in displacement of the cylinder which applies load to the load roller, all cause variations in the load applied which are not taken into account.

According to a preferred embodiment of the present invention these difficulties are avoided by measuring continuously both the applied load and the deflection produced by this load as a piece of timber passes through the roller system. Alternatively, a series of closely spaced, but discrete, measurements of applied load and the deflection produced by this load may be taken as a piece of timber passes through the roller system.

In one convenient arrangement a load cell is arranged to produce a signal proportional to the bending load applied by the load roller while a probe positioned opposite the load roller is arranged to operate a linear transducer which provides a signal proportional to deflection. By combining these signals to produce a resultant which is proportional to the quotient of bending load and deflection, such resultant will be a simple function of the modulus of elasticity. In this manner a much more accurate indication of the modulus is obtained than is possible when using any of the presently available grading machines.

In order that the invention may be better understood, two embodiments thereof will now be described by way of example only and with reference to the accompanying drawings in which:-

Figure 1 is a plan view of the central portion of a first embodiment of the machine of this invention;

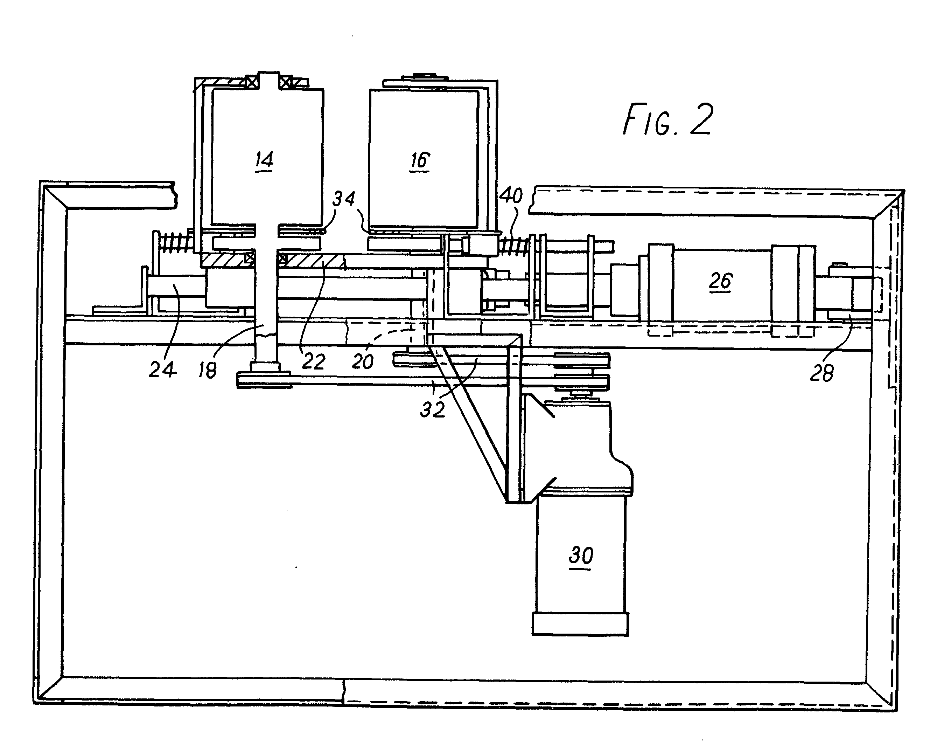

Figure 2 is a section of the line I-I in Figure 1; and

Figure 3 is a plan view of a second embodiment of the machine of this invention.

As shown the machine comprises a table 1 arranged at a convenient height on which is mounted two pairs of abutment rollers 2, 4 and 6, 8 respectively, these rollers being freely rotatable about fixed vertical axes. The spacing between the two pairs of abutment rollers determines the length of timber which is under test at any one moment. Conveniently this spacing is 90 cms. but to enable longer spans to be tested, which may be required in the case of timber with large cross sectional dimensions, the machine includes means which enables each pair of rollers to be mounted in another position on the table 1, for example where indicated at 20, 12 in respect of the rollers 2, 4.

Mounted on the machine centrally between the abutment rollers 2, 4 and 6, 8 is a pair of load rollers 14, 16 which are also rotatable about vertical axes. These rollers are mounted on shafts 18, 20 which are supported in bearings secured to a carriage 22, the carriage in turn being supported on slide rails 24 for movement transverse to the direction of movement of timber through the machine. The position of the carriage 22 is controlled by a pneumatic or hydraulic actuator 26 which is connected between the carriage and a bracket 28 fast with an extension of the table 2. A load cell, not shown, for measuring the pressure exerted by the actuator, is conveniently arranged between the actuator and the bracket 28.

The load rollers 14, 16 are each driven in the same direction of rotation by means of an electric motor 30 via belts 32, the motor being supported from

- 8 -                                    0037867

the carriage 22.

Each roller 14, 16 is formed at its lower end with a groove in which is located a deflection measuring probe 34.  Each probe is upported on respective slide rails 36, 38 which are fixed with the table 1 of the machine and each probe is biassed by springs 40 towards the central axis of the machine to a limiting position in which its periphery lies flush with the periphery of its associated load roller.  Outward movement of a probe from this limiting position is measured by an associated linear displacement transducer 42.

When the machine as thus far described is in its rest position with the carriage 22 positioned centrally the inner periphery of the abutment rollers 2 and 6 and the load roller 14 and its associated probe 34, and likewise the periphery of rollers 4, 8, 16 and associated probe 34, will lie on a straight line, the two lines being parallel and spaced apart by a distance somewhat greater than the maximum thickness of a piece of timber to be graded.  If now a piece of timber is positioned between the rollers and the carriage 22 is moved by the actuator 26 to the left, in the drawing, the load roller 16 will be pressed against one side of the timber while the other side will be pressed against the abutment rollers 2 and 6.  At the same time the probe 34 associated with load roller 14 will now extend proud of this roller and will be engaged by the timber.  As the pressure exerted by the actuator is increased the timber will be bent about the abutment rollers 2 and 6 and the extent of the deflection will be recorded by the resulting movement of the probe.  If on the other hand the actuator 26 is operated to move the carriage 22 to the right then the other load roller 14 will be effective to bend the timber about the other abutment rollers 4 and 8. If both load rollers are in rotation the timber will also be moved endwise through the machine in a direction determined by which load roller it is engaged.

During these operations the load cell associated with the actuator 26 will produce a signal which is proportional to the bending load applied to the timber while the transducer associated with the relevant probe will produce a signal which is proportional to the deflection produced by such load. These sign signals are combined to produce a resultant Q (load divided by deflection) which is a measure of the modulus of elasticity of the span of timber which is being deflected at that moment in time.

To control the operations of the machine it is provided with sensors (not shown) which detect the presence of timber at particular positions in the machine. A first such sensor may be positioned just in advance of the entry pair of abutment rollers (e.g. the rollers 2 and 4) while another such sensor may be positioned just beyond the other pair of abutment rollers (6 and 8). The two sensors together will detect the presence of a piece of timber spanning the two pairs of abutment rollers. The first sensor will detect the moment when the trailing end of the piece approaches the entry pair of rollers as the piece passes into the machine whilst the second sensor will detect the moment when the other, and now trailing, end of the piece approaches the other pair of abutment rollers as the piece repasses through the machine.

The machine may be provided with a number of rollers (not shown) which rotate about horizontal axes and on which the timber is supported during its passage through the machine. Such rollers may be driven or freely rotatable as desired. The machine may also be provided with pinch rolls which operate to feed timber into the machine and remove it therefrom.

The machine will be provided with a data processing system having a number of functions as will be described below. In part the operation of such system will be controlled by a pulse generator which is operated or synchronised by the rotation of the load rollers 14, 16 so as to provide a pulse at fixed intervals of linear motion of timber through the machine.

The cycle of operation is as follows:-

Before entry of a piece of timber the two lines of three rollers 2, 14, 6 and 4, 16, 8 are arranged so that their inner tangents are on straight lines. Pieces may freely enter the system by being dropped from above or by being projected into it by the rotation of driven horizontal rollers or pinch rolls, or by hand.

The fact that the piece has arrived in a position in which it spans the two pair of abutment rollers is detected by the two sensors and this causes the actuator 26 to move the load rollers 14, 16 on their carriage 22 in the direction which brings the forwarding feeding roller e.g. 16 and the probe 34 associated with the load roller 14 into contact with the timber. The pneumatic or hydraulic arrangements are such that this load roller delivers a suitable fixed non-destructive load approximately relative to the section of the timber.   Since this load need not be very accurately
fixed the arrangements may be simple and economical.

As the rotating load roller 16 presses the
timber against the opposing abutment rollers 2 and 6 it
will feed it through the machine until the trailing end
passes the first wood sensor.

During passage of the timber the indicating
parameter, Q, is repeatedly measured at fixed equal linear intervals, determined by the pulse generator.   It is
preferably digitalised and stored in a memory register of
the data processor.   When the trailing end of the timber
reaches the first wood sensor, this actuates a variable
time delay which is so set that, just before the trailing
end enters the leading abutment rollers 2, 4 the load
applying system reverses and presses the load roller
carriage 22 in the opposite direction bringing the face
of the opposite load roller 14 into contact with the
opposite side of the timber and by pressing it against the
other pair of abutment rollers 4, 8 bends the timber in
the opposite sense, bringing into action the opposite
deflection probe 34.   The timber is now passed in the
opposite direction from that on the first pass.   The
indicating output 'Q' is now derived as before and passes
to the data processor.   At each point as the timber
repasses, the data processor takes from the memory the
appropriate Q data caused by the passage of that particular span on the first pass and combines it with the
indicating figure obtained for the same span as it repasses in such a way that the resultant can be used as
the final indicator of modulus of elasticity of that
span.

This resultant is applied to one or more classifying thresholds and is thus classified or graded.

When the now trailing end of the timber passes the rollers 6, 8 the event is detected by the second sensor, or by the fall in load from the load cell, and this actuates the return of the load roller carriage 22 to its central position. The horizontal feed rollers or other means then carry the timber out of the machine.

The data processing system may incorporate a memory which will retain an indication of the lowest grading classification of any span in a piece of timber which has passed through the measuring system.

The machine may include a device or devices actuated by the data processing system for marking the timber according to its grading classification. Such devices may be operated to mark each span and/or to mark the timber as it leaves the machine according to the classification determined by the lowest grade span.

The improved machine may be modified in various ways. For example it could be operated by applying a fixed deflecting load to the load rollers 14, 16 and merely measuring the deflection produced. In such case the load cell could be omitted with a possible saving in cost but, as pointed out above, the grading may not be so reliable.

The machine may also be modified to provide a four point loading system instead of the three point loading system described. In such case the carriage 22 will be widened and arranged to carry two spaced pairs of load rollers which are each positioned near to and at a fixed distance from the adjacent pair of abutment rollers. The actuator for moving the carriage and the associated load cell could be arranged as described while the deflection probes and associated transducers would be positioned so as to measure deflection at the centre of the span under test and possibly mounted on the carriage.

It will be appreciated that the data processing system may be varied or elaborated according to the requirements. For example it may also be arranged to determine the average grade of each span of the timber being graded and to cause the timber to be marked to indicate such grade. The system may also be arranged to display the grade of each span as measured, the grade of the weakest span and also the average grade and the system may also display the applied load and resulting deflection. The system could also include memories which may record such data for all the pieces of timber which have been graded so that suitable statistical information may be provided.

A further advantage of a machine in which both deflection and load are measured and the resulting quotient used as the control parameter is that it makes possible a very fast and accurate stress grading machine.

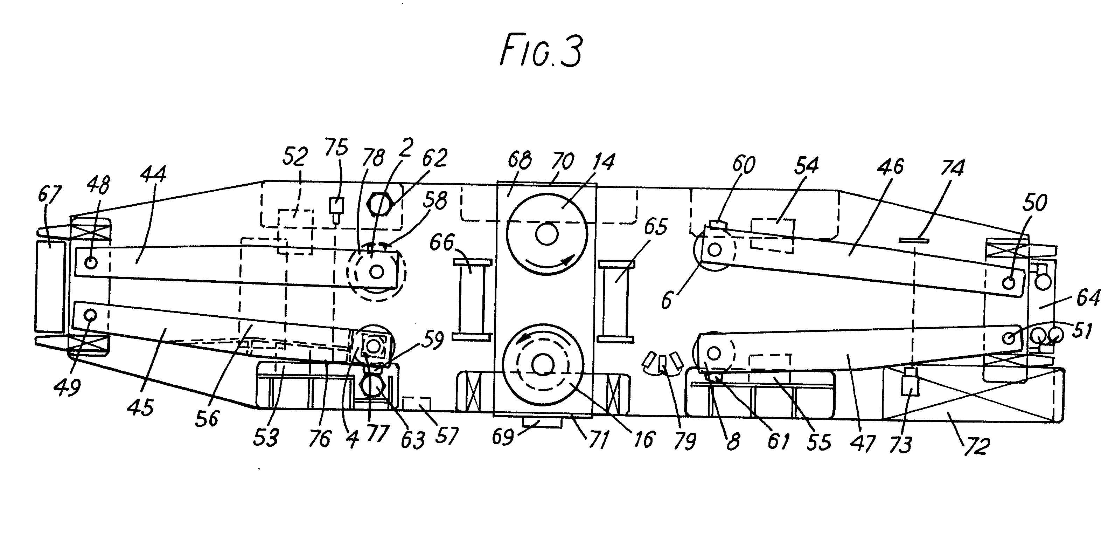

A second embodiment of a machine according to the invention is shown in Figure 3 to which reference will now be made. The machine comprises two pairs of freely rotatable abutment rollers 2,4 and 6,8 together with a pair of load rollers arranged in a similar manner to that above described. The load rollers are selectively drivable by drive means (not shown) in the directions indicated by the arrows A, B.

The major difference between this embodiment and that described previously is the manner in which the various rollers are used to bend the timber as it passes through the machine in the direction indicated. In this embodiment the vertical axes of the drive rollers are fixed, and it is the abutment rollers that move to bend the timber. For this purpose, each abutment roller 2, 4, 6, 8, is mounted on the free end of a respective support arm 44, 45, 46, 47 pivotted about a respective pivot 48, 49, 50, 51. Movement of each arm about its respective vertical axis is effected by means of respective pneumatically operated bellows 52, 53, 54 and 55, supplied from an air receiver 56 via automatically controlled air valves 57. Movement of the support arms and their associated rollers in a direction away from the path of timber is limited by means of respective abutment stops 58, 59, 60, 61. The stops 58 and 59 are adjustable in position by means of respective deflection setting cams 62, 63. Timber passing through the machine is supported by idler rollers 64, 65, 66 and 67.

The load rollers 14, 16 are mounted on a central carriage 68 which is movable in a lateral direction only to apply pressure, during use of the machine, to a load measuring transducer 69. For this purpose the carriage is mounted very stiffly, for a limited amount of lateral movement, between respective carriage diaphragms 70, 71.

Passage of timber through the machine is under the control of a small dedicated computer 72 which receives positional information about the timber passing through the machine from an entry photoelectric switch 73 and associated reflector 74, and an exit photoelectric switch 75 and its associated reflector 76. Information as to the speed of passage of timber through the machine is fed to the computer 72 from a speed encoder 77.

The sequence of operation will now be explained. First, the cams 62 and 63 are adjusted by hand to the deflection stipulated for the specific size and species of timber to be tested. A range of six preset deflection settings are available. The air pressure to be applied to the bellows 54, 55, 56 and 57 is set at a suitable level for the timber concerned - too great a pressure may result in damage to the timber due to crushing, and can reduce the effective drive on the timber. In the particular embodiment under discussion, two distinct pressure levels are available - high and low - but more levels could be incorporated. Finally, the speed at which timber is to be driven through the machine is set - a typical range of speeds is 25 to 100 linear metres/second.

Timber to be tested is now inserted into the machine at the right hand end and is moved leftwards, by hand, over the idler rollers 64 to 67. The narrowest edge of the timber rests on the rollers. The bellows 52 and 53 are initially set so that, for the particular size of timber being tested, the gap between the abutment rollers 2, 4 is slightly less than the width of the timber and is disposed symmetrically about the centre line of the machine. The rollers 2, 4 act to nip the timber so that further movement of the timber starts to rotate the rollers 2 and 4. Rotation of roller 4 also causes the speed encoder 77 to commence operation and the computer is programmed such that, after a predetermined length of timber has passed between rollers 2 and 4, the computer takes over all further control of the movement of the timber. This is achieved by applying air to a pneumatic brake 78 associated with the roller 2 which presents the operator with a sudden high resistive load which signals him to let go of the timber.

After a preset time delay, air is admitted at a controlled rate to the bellows 52 and 54, the air brake 78 remaining on and the bellows 53 and 55 being ported to exhaust. During this time the timber in the machine is progressively bent about the entry drive and load roller 16 due to pressure from the abutment rollers 2 and 6 against the timber. This causes a reaction force which is measured by the load transducer 69. Also during this time, the load rollers are revolving in the direction indicated by the arrows, and attempting to drive the timber through the machine.

When the reaction force as measured by the transducer 69 reaches a predetermined level, set in accordance with the timber being tested, the air brake 78 is released and the timber allowed to accelerate through the machine. The bellows 52 and 54 continue to apply a bending force to the timber and when the stops 59, 61 reach their respective abutments, an electrical signal is sent to the computer and readings given by the

0037867

encoder 77 and the load transducer 69 at this time are stored by the computer.

In the case of exceptionally strong timber, the computer will start logging data from the encoder 77 and transducer 69 without waiting for the stops 59 and 61 to contact their respective abutments. This is achieved by continuing to monitor the output from the transducer and, if this should exceed a predetermined value before the stops contact the abutments, then continuous logging is commenced.

As the timber continues its driven leftwards movement, the leading edge of the timber will pass between the light switch 75 and its associated reflector 76, thus breaking the beam. The computer registers this as the timber datum edge and sets a reference point in its memory to which all previous logged data is related. The computer also sets a "load off" marker in one of its registers for use during the second cycle of timber. This "load-off" marker reprsents a preset distance from the leading edge of the timber.

The timber continues its leftwards motion through the machine, and the computer logs the output of the transducer 69 at a predetermined pitch along the length of the timber. Eventually, the trailing edge of the timber clears the light beam passing between light switch 73 and its associated reflector 74. This signals the computer to start counting pulses from the output of encoder 77 and, after a predetermined number of such pulses, to apply air to the air brake 78. At the same time the output from transducer 69 ceases to be logged; however, the computer continues to take information from the encoder 77 so that the deceleration of the timber can be logged.

Simultaneous with the reconnection of the light beam as the trailing edge of the timber passes the light switch 73, air is ported to bellows 53 and 55 and bellows 52 and 54 are ported to exhaust. It should be noted that it is desirable that the exhaust arrangements of bellows 52 and 54 are independent of one another to ensure that the travel of the abutment roller 6 lags that of roller 8, thereby reducing vibration at the leading end of the timber.

The whole procedure is now reversed, with bellows 53 and 55 continuing to apply a bending force to the timber over the load roller 14 until the stops 58, 60 contact their respective abutments. As before, readings from the encoder 77 and transducer 69 are logged in the computer as the timber passes back through the machine, driven by roller 14. When the originally trailing, now leading, edge of the timber once again breaks the light to light switch 73, the computer checks the registration of the information stored in its memory from the encoder 77 against that taken during the first pass. An error higher than a preset amount will cause the computer to abort the grading operation.

The timber continues through the machine in the rightwards direction, encoder and transducer readings being taken at regular intervals, as described above. Grading measurements continue until the "load-off" marker, previously placed in the computers memory- see above, is reached, at which point measurements cease.

The computer then calculates the average as between corresponding readings on the forward and reverse passes and causes the timber to be sprayed automatically with the appropriate grade.  This may be achieved by appropriate actuation of a group of spray guns 79.

The bellows 53 and 55 are quickly ported to exhaust which immediately releases the timber from the pinch condition at both sets of abutment rollers. The timber them continues rightwards movement under its own inertia out of the machine.  If required an end marking unit 80 may be actuated as the timber leaves the machine to apply the appropriate stamp.

If desired a third "timber sorting" pass may be provided for.  Acting on the information initially entered about the timber into the computer and after the grading operation has ceased, the air brake 48 is applied <u>before</u> the bellows 53 and 55 are ported to exhaust.  Air is thence ported to bellows 52 and 54 and bellows 53 and 55 are ported to exhaust.  The timber is driven back through the machine in the leftwards direction and allowed to proceed under its own inertia after the light beam of light switch 73 is cleared.

0037867

<u>CLAIMS</u>

1            A method of stress grading timber, said method comprising passing timber to be graded in a forward direction through a system of rollers whilst simultaneously causing said rollers to bend the timber in one direction, thence reversing the timber so that it passes back through the rollers in the reverse direction whilst simultaneously causing said rollers to bend the timber in the other direction, and monitoring the conditions under which the timber is bent so as to enable the timber to be graded.

2            A method as claimed in claim 1 wherein said rollers bend the timber by passing the timber across two spaced abutment rollers, and applying a bending force to the timber passing between said abutment rollers by means of a load roller positioned between the abutment rollers, and acting on the timber in such a way as to force the timber against the abutment rollers.

3            A method as claimed in claim 2 wherein the roller system comprises two spaced pairs of said abutment rollers and arranged centrally between them a pair of said load rollers, and wherein the timber on its first, forward, traverse is passed between a first of said pairs of abutment rollers, then between the load rollers and then between the second of said pair of abutment rollers, the point at which the trailing end of the timber approaches the vicinity of said first pair of rollers being sensed, whereupon the timber is stopped, reversed, and passed back through the rollers on a reverse traverse.

4            A method as claimed in either one of claims 2 or 3 further including sensing the passage of the timber as its leading edge, on the first traverse, passes beyond the trailing pair of abutment rollers and as its trailing edge, on the reverse traverse, passes into the same pair of abutment rollers.

5           A method as claimed in any one of the preceding claims wherein the conditions under which the timber is bent are monitored by measuring continuously or at spaced intervals both the load applied to bend the timber and the deflection produced by this load during passage of the timber through the roller system.

6           A method as claimed in any one of claims 1 to 4 wherein the conditions under which the timber is bent are monitored by applying a fixed load to bend the timber during its passage through the roller system and measuring the deflection produced by this load.

7           A method as claimed in any one of claims 1 to 4 wherein the conditions under which the timber is bent are monitored by bending the timber by a fixed amount during its passage through the roller system and measuring the load required to produce this amount of bending.

8           A method of stress grading timber as claimed in claim 1, substantially as hereinbefore described.

9           Apparatus for the stress grading of timber, said apparatus comprising a series of rollers through which timber to be tested may pass, reversible drive means for moving the timber through the roller system, means for reversing the drive of said drive means after the timber has passed or almost passed through the roller system in a forward direction, means for causing said rollers to bend the timber in one direction as it passes through the roller system in the forward direction and in the opposite direction as the timber passes through the roller system in the reverse direction, and means for monitoring the conditions under which the timber is bent so as to enable the timber to be graded.

10  Apparatus as claimed in claim 9 wherein the roller system comprises a minimum of six rollers arranged in two sets of three, each set comprising a pair of spaced abutment rollers and a load roller situated centrally between them, but arranged to be on the opposite side of the timber, when present, and wherein means are provided whereby the pair of load rollers an the one hand and the two pairs of abutment rollers on the other hand may be moved relatively to one another in a direction transverse to the direction of movement of the timber under test in such a manner that while the timber is passing through the machine in one direction one of the two load rollers presses the timber against and bends it between the opposed abutment rollers whereas when the timber is passing through the machine in the opposite direction it is the other load roller and the other two abutment rollers which act to bend the timber.

11  Apparatus as claimed in claim 10 wherein the two load rollers are mounted in a carriage which is movable in the required direction under control of a hydraulic or pneumatic actuator, while the abutment rollers are held fixed.

12  Apparatus as claimed in claim 10 wherein the axes of the load rollers are fixed while the abutment rollers are moved each by a respective hydraulic or pneumatic actuator transversely of the direction of movement of the timber, the extent of movement away from the path of the timber being limited by means of stops.

13  Apparatus as claimed in any one of claims 10 to 12 wherein the drive means comprises means for rotating one or more of the rollers in said roller system.

14       Apparatus as claimed in claim 13 wherein the two load rollers are driven, and the four abutment rollers are freely rotatable.

15       Apparatus as claimed in claim 13 wherein at least one of the abutment rollers are driven, and the load rollers are freely rotatable.

16       Apparatus as claimed in any one of claims 10 to 15 further including sensor means positioned so as to be able to cause the drive means to halt and reverse when the trailing end of the timber entering the machine approaches the vicinity of the leading pair of abutment rollers.

17       Apparatus as claimed in any one of claims 10 to 16 further including further sensor means positioned just beyond the trailing pair of abutment rollers, in the direction of the first traverse of timber through the apparatus, said further sensor being operable to detect the passage of the leading end of the timber, on the first traverse, to initiate operation of the apparatus and to detect the passage of the trailing edge of the timber, on the reverse traverse, to halt operation of the apparatus.

FIG. 1

FIG. 2

0037867

FIG.3

European Patent Office

EUROPEAN SEARCH REPORT

0037867
Application number

EP 80 30 1193.1

| | DOCUMENTS CONSIDERED TO BE RELEVANT | | CLASSIFICATION OF THE APPLICATION (Int. Cl.³) |
|---|---|---|---|
| Category | Citation of document with indication, where appropriate, of relevant passages | Relevant to claim | |
| | GB - A - 1 104 986 (H.E. BOOTH et al.)<br>* claim 1, page 2, lines 4 to 10; fig. 1 * | 1 | G 01 N 33/46<br>G 01 N 3/20 |
| A | GB - A - 1 322 953 (COOK BOLINDERS LTD.)<br>-- | | |
| A | US - A - 3 196 672 (H.A. KELLER)<br>---- | | |
| | | | TECHNICAL FIELDS SEARCHED (Int.Cl.³) |
| | | | G 01 N 3/20<br>G 01 N 33/46 |
| | | | CATEGORY OF CITED DOCUMENTS |
| | | | X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| X | The present search report has been drawn up for all claims | | &: member of the same patent family, corresponding document |

| Place of search | Date of completion of the search | Examiner |
|---|---|---|
| Berlin | 28-11-1980 | SCHWARTZ |

EPO Form 1503.1   06.78